# EUROPEAN PATENT SPECIFICATION

(11) **EP 1 413 332 B1**
(45) Date of publication and mention of the grant of the patent: **31.12.2008**
(21) Application number: 02745439.6
(22) Date of filing: 01.07.2002
(51) Int. Cl.: A61K 45/06, A61K 31/185, A61K 31/519, A61K 33/26, A61P 15/10

(54) **USE OF 2,5-DIHYDROXYBENZENESULPHONIC ACID DERIVATIVES IN THE PRODUCTION OF A MEDICAMENT USED TO POTENTIATE THE EFFECT OF OTHER DRUGS IN THE TREATMENT OF ERECTILE DYSFUNCTION**
VERWENDUNG VON 2,5-DIHYDROXYBENZOLSULFONSÄURE-DERIVATEN BEI DER HERSTELLUNG EINES MEDIKAMENTS ZUR VERSTÄRKUNG DER WIRKUNG ANDERER ARZNEIMITTEL ZUR BEHANDLUNG DER EREKTILEN DYSFUNKTION
UTILISATION DE DERIVES D'ACIDES 2,5-DIHYDROXYBENZENOSULFONIQUES DANS L'ELABORATION D'UN MEDICAMENT POUR STIMULER L'EFFET D'AUTRES SUBSTANCES PHARMACEUTIQUES DANS LE TRAITEMENT DU DYSFONCTIONNEMENT ERECTILE

(30) Priority: 02.07.2001 ES 200101535
(43) Date of publication of application: 28.04.2004
(73) Proprietor: LABORATORIOS DEL DR. ESTEVE, S.A., 08041 Barcelona (ES)
(72) Inventor: ESTEVE-SOLER, José, E-08041 Barcelona (ES); SAENZ DE TEJADA-GORMAN, Inigo, E-08041 Barcelona (ES)
(74) Representative: Carpintero Lopez, Francisco
(86) International application number: PCT/ES2002/000325
(87) International publication number: WO 2003/004097

(56) References cited:
- WO-A-97/37647
- WO-A-98/29103
- FR-A- 2 511 598
- US-A- 3 681 503
- US-A- 4 252 821
- DATABASE WPI Derwent Publications Ltd., London, GB; AN 1993-402392, XP002963114 & SU 1 776 408 A (ODESS PIROGOC INST) 23 November 1992

## Description

### Field of the Invention

The present invention refers to the use of calcium 2,5-dihydroxybenzene sulphonate for the manufacture of a medicament to enhance the effects of sildenafil or sodium nitropressiate to facilitate penile erection in man. Further disclosed is the use of 2,5-dihydroxybenzenosulphonic acids of general formula (I) in the production of medicinal products of therapeutic value to enhance the effects of phosphodiesterase-5 inhibitors including sildenafil, vardenafil and IC-351, nitric acid donors including amyl nitrate, nitroglycerine, nitroprussiate, nitrosothiols and nicorandyl, of compounds that increase cyclic GMP levels in the penile tissue and of other compounds used to facilitate penile erection in man.

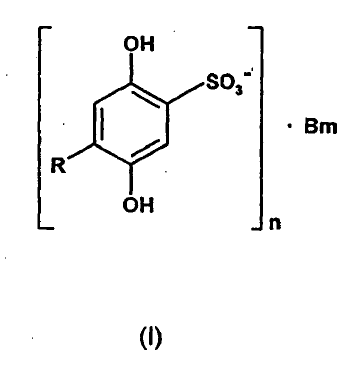

### Detailed description of the invention

The present invention refers to the use of calcium 2,5-dihydroxybenzene sulphonate for the manufacture of a medicament to enhance the effects of sildenafil or sodium nitropressiate to facilitate penile erection in man. WO 97/37647 discloses the use of 2,5-dihydroxybenzenesulphonic acid derivatives, particularly calcium 2,5 dihydroxybenzenesulphonate (calcium dobesilate) for the treatment of erectile dysfunction.

In recent studies, we have shown that calcium 2,5-dihydroxybenzene sulphonate exerts effects on the resistance arteries of the human penis that result in enhancement of the effect of the phosphodiesterase-5 inhibitor, sildenafil, and of the nitric acid donor sodium nitropressiate to facilitate penile erection in man.

It is known that the therapeutic response to sildenafil is variable in different patients and often does not exceed 50% [MS Rendell et al, JAMA 1999, 281: 421-426; R Virag, Urology 1999; 54: 1073-1077], which creates a deficient therapeutic situation.

The compounds referred to in the present invention fit in the general formula (I): 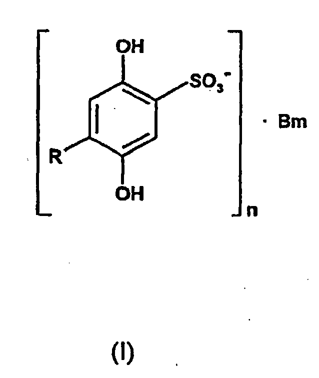 in which:
R represents a hydrogen atom or a sulphonate group (SO₃⁻);
B represents a calcium ion (Ca⁺⁺) or a diethylammonium group [H₂N⁺(C₂H₅)₂];
n represents 1 or 2; and
m represents 1 or 2.

The compounds of the following examples are prepared according to the procedures described previously:

### Example 1

Calcium 2,5-dihydroxybenzenesulphonate (Calcium dobesilate). "The Merck Index", 12 edition, Merck & Co., Whitehorse Station, N.J., USA, 1996.

### Reference Example 2

Diethylammonium 2,5-dihydroxybenzenesulphonate (Ethamsilate). "The Merck Index", 12 edition, Merck & Co., Whitehouse Station, N.J., USA, 1996.

### Reference Example 3

Bis-diethylammonium 2,5-dihidroxybenzene-1,4-disulphonate (Bis-diethylammonium persilate). French patent FR 73/17709 (publication number 2.201.888).

To study the enhancing effect of medicinal products used to facilitate penile erection in man a series of studies were carried out of the resistance arteries of the human penis, obtained from patients submitted to penile prosthesis implantation.

Specimens of human cavernous bodies of the penis were obtained from patients with impotence while these were intervened for prosthetic implantation, as described previously (Gupta et al.; Br. J. Pharmacol., 116: 2201, 1995). The tissues were deposited in M-400 solution (pH 7.4; 400 mOsm/kg. Composition in w/v: 4.19% manitole, 0.2% KH₂PO₄, 0.97% K₂HPO₄·3 H₂O, 0.11% KCI and 0.08% NaHCO₃) at 4°C at the moment of explant and were transported to the laboratory to be used within the following 16 h.

The resistance arteries of the penis, helicine arteries (with a luminal diameter of 150-400 µm), which are terminal branches of the deep arteries of the penis, were dissected carefully removing the surrounding trabecular tissue and were cut into 2 mm long arterial segments that were arranged on two wires of 40 µm diameter in a Halpern-Mulvany myograph (J.P. Trading, Aarhus, Denmark) to record isometric pressure. The cavities contained physiological saline solution (PSS) through which a mixture of 95% O₂/5% CO₂ was continually passed to maintain this oxygenated and to maintain the pH at around 7.4. The arteries were contracted with 1 µM of noradrenaline and their relaxation responses were assessed after adding to the cavities increasing amounts of the different compounds. Transmural electrical stimulation (TES) was carried out using two electrodes placed parallely to the arterial segment and connected to a stimulator with a direct output current (50 mA). Squared pulses were applied of 0.3 ms duration in relays of 15 s with variable frequency (0.5, 1, 2 and 6 Hz).

### Effects on the relaxation of resistance arteries of the human penis enhanced by a specific nitric oxide donor.

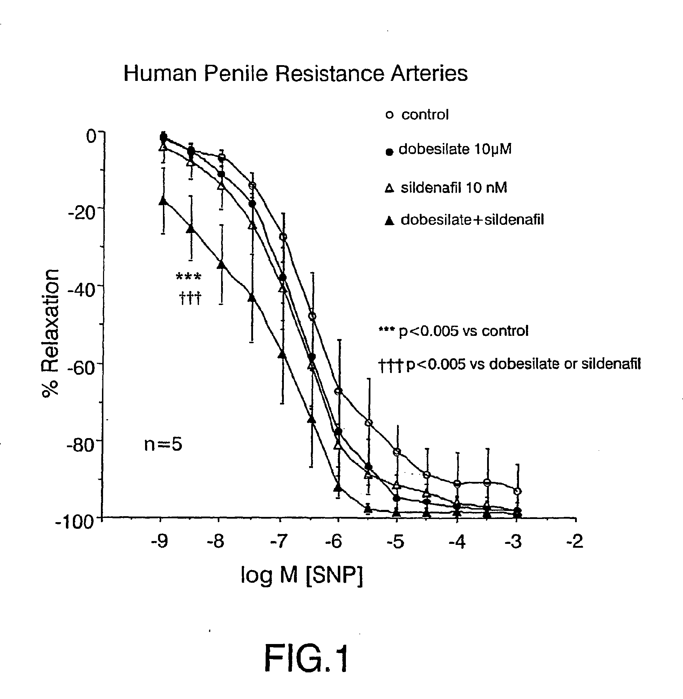

Calcium dobesilate at a concentration of 10µM increases, in a statistically significant manner, the relaxation produced by different concentrations of sodium nitroprussiate (SNP), a known nitric oxide donor (fig 1).

### Effects on the relaxation of resistance arteries of the human penis induced by sildenafil.

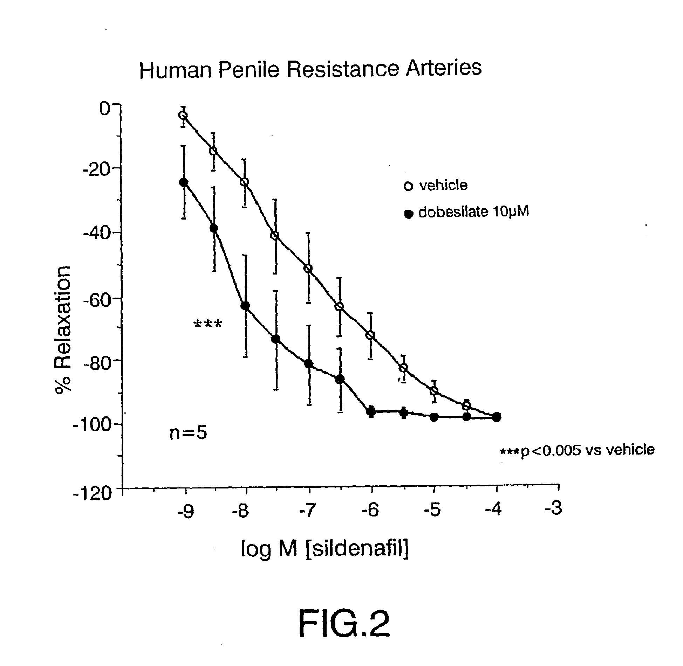

Calcium dobesilate at a concentration of 10 µM increases, in a statistically significant manner, the relaxation produced by different concentrations of the inhibitor of 5-sidenafil phospodiesterase (fig. 2).

### Effects on the relaxation of resistance arteries of the human penis induced by electrical stimulation of nitrergic terminations.

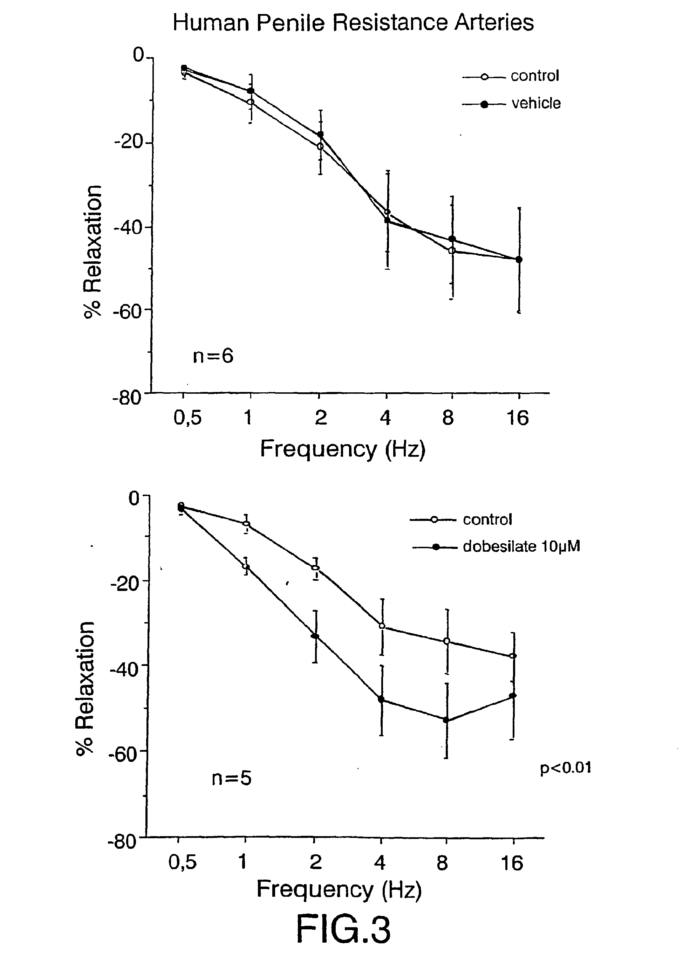

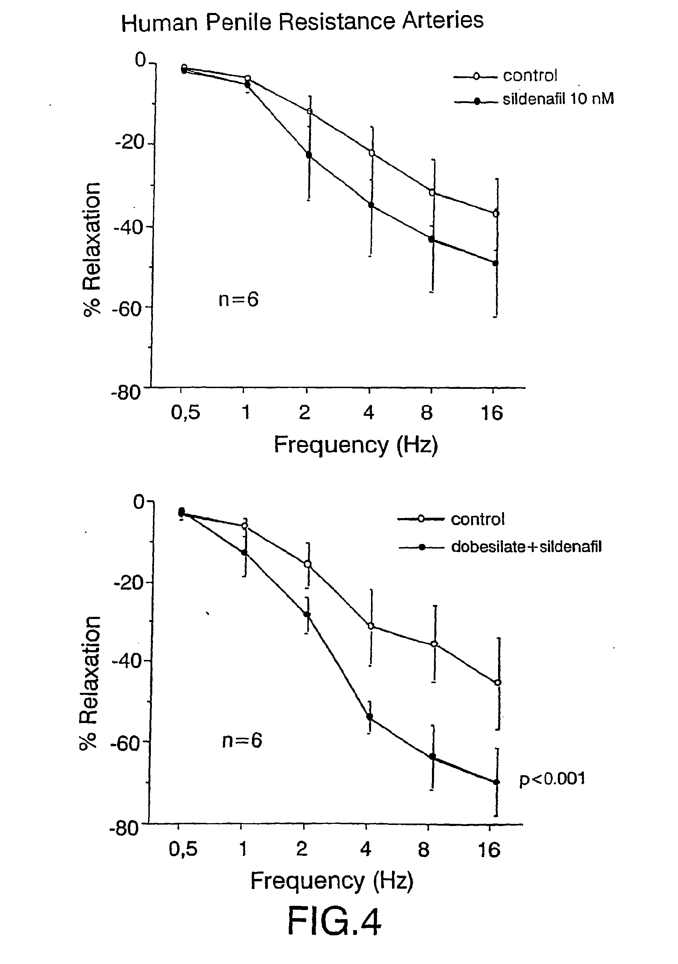

Calcium dobesilate at a concentration of 10 µM increases, in a statistically significant manner, the relaxation produced by electrical stimulation at increasing frequencies of the nitrergic terminations in resistance arteries of the human penis (fig 3). This effect is similar and even greater than that produced by sildenafil at a concentration of 10 nM (fig 4).

Calcium dobesilate, at a concentration of 10 µM, increases, in a statistically significant manner, the effects of 10 nM of sildenafil on the relaxation produced by electrical stimulation at increasing frequencies of the nitrergic terminations in resistance arteries of the human penis (fig 4).

## Claims

1. The use of calcium 2,5-dihydroxybenzenesulphonate for the manufacture of a medicament to enhance the effects of sildenafil to facilitate penile erection in man.

2. The use of calcium 2,5-dihydroxybenzenesulphonate for the manufacture of a medicament to enhance the effects of sodium nitroprussiate to facilitate penile erection in man.

## Patentansprüche

1. Verwendung von Calcium-2,5-dihydroxybenzolsulfonat zur Herstellung eines Medikaments zur Verstärkung der Wirkungen von Sildenafil bezüglich der Erleichterung der Peniserektion beim Mann.

2. Verwendung von Calcium-2,5-dihydroxybenzolsulfonat zur Herstellung eines Medikaments zur Verstärkung der Wirkungen von Natriumnitroprussiat bezüglich der Erleichterung der Peniserektion beim Mann.

## Revendications

1. Utilisation de calcium 2,5-dihydroxybenzènesulfonate pour la fabrication d'un médicament pour renforcer les effets du sildénafil pour faciliter l'érection pénienne de l'homme.

2. Utilisation de calcium 2,5-dihydroxybenzènesulfonate pour la fabrication d'un médicament pour renforcer les effets du nitroprussiate de sodium pour faciliter l'érection pénienne de l'homme.
